# EUROPEAN PATENT APPLICATION

(11) **EP 4 112 034 A1**
(43) Date of publication of application: **04.01.2023**
(21) Application number: 21183028.6
(22) Date of filing: 01.07.2021
(51) Int. Cl.: A61J 1/00, A61J 7/04, G16H 20/13

(54) **MEDICINE PILLBOX FOR MANAGING MEDICINE CONSUMPTION**

(71) Applicant: IP Full Asset Limited, Hong Kong (HK)
(72) Inventor: The designation of the inventor has not yet been filed
(74) Representative: Meyer-Dulheuer MD Legal Patentanwälte PartG mbB

(57) **Abstract**

A technique is provided for managing medical consumption. The technique includes a micro controller unit (MCU), a transceiver communicatively coupled to an electronic device, plurality of compartments for storing a plurality of medicines, and a memory. The MCU automatically generates an indicia associated with each of the plurality of compartments. Each of the plurality of compartments is assigned unique generated indicia. Further, the indicia is transmitted to a pharmacy server along with medical prescription data. A label of each of the unique generated indicia is affixed to a packaging of each of a plurality of medicines as provided in the medical prescription. Each of the plurality of medicines is stored in corresponding plurality of compartments having the same unique generated indicia, and each compartment stores only one type of medicine from the plurality of medicines. A reminder alert is generated for indicating a time for consuming the plurality of medicines.

## Description

### Technical Field

This disclosure relates generally to a medicine pillbox, and more particularly to method and device for managing medicine consumption using a medicine pillbox.

### Background

The change of lifestyles in the current century has not only burdened the generation with new medical issues, but has also made the recovery process all the more difficult. This is partly because of the busy lifestyles led by patients that lead to deviation of the patients from the medicine dosage schedule prescribed to them by the medical practitioners. In certain other scenarios, following the medicine dosage schedule may be difficult owing to various other indispositions of the patients, such as age, mental condition, and the like.

Presently, various solutions exist to offer assistance to the patients in sticking the medicine dosage schedule and avoiding the possibility of missing out on consuming prescribed dosages of medicine while following a treatment course. One such solution relies on a medicine receptacle for storing medicine dosages that are to be consumed at various times in a day. Each receptacle includes a unique identification that may be used by a mobile-based application for correctly advising the patient concerning dosage strength and dosage frequency for consuming one or more medicines stored in a receptacle. The mobile device may further advise the user by means of a wearable by displaying one or more unique identification of the receptacles on the wearable.

In another scenario, a pillbox having a plurality of compartments, each corresponding to a dose to be taken by a patient at a designated time during a day, has been proposed. The pillbox may be integrated with a mobile application that reminds and assists the patient in identifying one or more medicines that are to be consumed at a given time in a day and record the consumption thereof.

However, both the existing solutions fail to address issues relating to tracking of the quantity of a given medicine within a receptacle or a compartment of the pillbox. Specifically, because a compartment of the pillbox in the second solution stored medicines belonging to different types, it is exceedingly difficult to track the quantities of a particular medicine in the compartment, thereby leading to a probable running out of stock. Further, the present solutions rely on the judgement of the patient in correctly identifying a type of medicine thereby leading to possible errors in consumption of correct medicines. Furthermore, the present solutions put the burden of initial arrangement of the medicines in correct receptacles and compartment on the patient, which can be cumbersome.

Therefore, there exists a need to provide a system and/or method that facilitates seamless integration of a pillbox and peripheral devices that assist a patient in efficiently managing medicine consumption. Further, there is a need for a method and system that offloads the patient from the burden of having arranging the medicines in correct order in various compartments and of tracking the quantities of medicines available.

Further limitations and disadvantages of conventional and traditional approaches will become apparent to one of skill in the art, through comparison of described systems with some aspects of the present disclosure, as set forth in the remainder of the present application and with reference to the drawings.

### SUMMARY

In one embodiment, a medicine pillbox for managing medicine consumption is disclosed. The medicine pillbox includes a micro controller unit (MCU), a transceiver installed within the medicine pillbox and configured to communicate with an electronic device, a plurality of compartments configured to store a plurality of medicines, and a memory communicatively coupled to the MCU. The memory may be configured to store MCU instructions, which, on execution, cause the MCU to automatically generate an indicium associated with each of the plurality of compartments. Each of the plurality of compartments may be assigned the unique generated indicia. The instructions further cause the processor to transmit the generated indicia to a pharmacy server along with medical prescription data, such that a label of each of the unique generated indicia may be affixed to a packaging of each of a plurality of medicines as provided in the medical prescription. Further, each of the plurality of medicines may be stored in each of the corresponding plurality of compartments having the same unique generated indicia and each compartment from the plurality of compartments stores only one type of medicine from the plurality of medicines. The instructions further cause the processor to generate a reminder alert indicating a time when a patient is to consume the plurality of medicines.

In another embodiment, an electronic device for managing medicine consumption is disclosed. In one example, the electronic device includes a processor and a memory communicatively coupled to the processor. The memory stores processor instructions, which, on execution, causes the processor to receive an input via Graphical User Interface (GUI) from a patient to book an order of a plurality of medicines based on medical prescription data. The processor instructions further cause the processor to transmit the order to an online pharmacy store server for procuring and dispatching the plurality of medicines to the patient. An automatically generated unique indicia may be allocated to each of the plurality of medicines and a label of each of the unique generated indicia may be affixed to a packaging of each of a plurality of medicines as provided in the medical prescription data. The processor instructions further cause the processor to provide a reminder notification to the patient to consume the plurality of medicines based on the medical prescription data.

In yet another embodiment, a method for managing medicine consumption using a medicine pillbox and an electronic device, is disclosed. The method includes a micro controller unit (MCU) that automatically generates an indicium associated with each of the plurality of compartments of the medicine pillbox. Each of the plurality of compartments may be assigned the unique generated indicia. The method further includes transmitting the generated indicia to an online pharmacy store server along with medical prescription data. A label of each of the unique generated indicia may be affixed to a packaging of each of a plurality of medicines as provided in the medical prescription and each of the plurality of medicines may be stored in each of the corresponding plurality of compartments having the same unique generated indicia. Further, each compartment from the plurality of compartments stores only one type of medicine from the plurality of medicines. The method further includes generating a reminder alert indicating a time when a patient is to consume the plurality of medicines. The method further includes displaying the unique indicia associated with the medicine to be consumed at time instant as prescribed in the medical prescription data.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this disclosure, illustrate exemplary embodiments and, together with the description, serve to explain the disclosed principles.
FIG. 1 is a block diagram of an exemplary network environment for managing medicine consumption using a medicine pillbox and an electronic device, in accordance with some embodiments of the present disclosure.
FIG. 2 is a block diagram of exemplary medicine pillbox for managing medicine consumption, in accordance with some embodiments of the present disclosure.
FIG. 3 is a block diagram of exemplary electronic device for managing medicine consumption, in accordance with some embodiments of the present disclosure.
FIG. 4 illustrates an exemplary scenario for managing medicine consumption using a medical pillbox and an electronic device, in accordance with some embodiments of the present disclosure.
FIG. 5 is a flow diagram of a detailed exemplary process for managing medicine consumption using a medical pillbox and an electronic device, in accordance with some embodiments of the present disclosure.
FIG. 6 is a flow diagram of a detailed exemplary process for generating indicia associated with plurality of compartments of a medicine pillbox for managing medicine consumption, in accordance with some embodiments of the present disclosure.

### DETAILED DESCRIPTION

Exemplary embodiments are described with reference to the accompanying drawings. Wherever convenient, the same reference numbers are used throughout the drawings to refer to the same or like parts. While examples and features of disclosed principles are described herein, modifications, adaptations, and other implementations are possible without departing from the spirit and scope of the disclosed embodiments. It is intended that the following detailed description be considered as exemplary only, with the true scope and spirit being indicated by the following claims.

Various implementations may be found in a method and/or a system for managing medicine consumption using a medicine pillbox and an electronic device, in accordance with some embodiments of the present disclosure. The medicine pillbox proposed herein includes a micro controller unit (MCU), a transceiver installed within the medicine pillbox and configured to communicate with an electronic device, a plurality of compartments configured to store a plurality of medicines, and a memory communicatively coupled to the MCU. The memory may be configured to store MCU instructions, which, on execution, cause the MCU to automatically generate an indicia associated with each of the plurality of compartments. Each of the plurality of compartments may be assigned the unique generated indicia.

In an embodiment, each of the plurality of compartments has a plurality of openable lids. In another embodiment, the medicine pillbox has an openable lid that covers each of the plurality of compartments.

In an embodiment, automatically generating the indicia includes generating a sequential number starting from '1' up to 'N' based on a number of the plurality of compartments. In another embodiment, the automatically generated indicia may have an associated unique colour.

In an embodiment, the automatically generated indicia may be displayed on each of the plurality of compartments. The instructions further cause the processor to transmit the generated indicia to a pharmacy server along with medical prescription data, such that a label of each of the unique generated indicia may be affixed to a packaging of each of a plurality of medicines as provided in the medical prescription. In another embodiment, pharmacy personnel may affix a label for each of the unique generated indicia with an associated unique colour or sequential numbers, to a packaging of each of a plurality of medicines as provided in the medical prescription.

In an embodiment, the medical prescription data includes information about quantity of each of the medicines, dosage of the medicines, and a time at which the prescribed medication should be consumed. Further, the medical prescription may be received by the medicine pillbox from a doctor.

Further, each of the plurality of medicines may be stored in each of the corresponding plurality of compartments having the same unique generated indicia and each compartment from the plurality of compartments stores only one type of medicine from the plurality of medicines. In an embodiment, the patient sorts each of the plurality of medicines in the corresponding compartment by matching the label affixed on the packaging of each of a plurality of medicines with the indicia displayed on each of the plurality of compartments. Further, the instructions further cause the processor to generate a reminder alert indicating a time when a patient is to consume the plurality of medicines.

In an embodiment, automatically generating the indicia associated with each of the plurality of compartments further includes identifying if one or more medicines from the plurality of medicines has been discontinued based on a user input or prescription data received via the electronic device. The generation further includes based on such identification, releasing the indicia allocated to the one or more medicines for future allocation. Further, when a user orders a new medicine then the released indicia may be assigned to the new medicine. Furthermore, such an indicia reassignment may be displayed on the electronic device of the patient and the patient may be asked to confirm the new assignment or edit the indicia assigned to the new medicine.

In an embodiment, a weight sensor may be associated with each of the plurality of compartments. The MCU may be configured to automatically place an order at a pharmacy store server for one or more medicines from the plurality of medicines based on the medical prescription data (particularly including the quantities that have been prescribed) and the information received from the weight sensor. In another embodiment, the MCU may be configured to automatically place an order at the pharmacy store server for one or more medicines from the plurality of medicines based on the medical prescription data and after a pre-defined time interval.

In yet another embodiment, an electronic device for managing medicine consumption, is disclosed. In one example, the electronic device includes a processor and a memory communicatively coupled to the processor. The memory stores processor instructions, which, on execution, causes the processor to receive an input via Graphical User Interface (GUI) from a patient to book an order of a plurality of medicines based on medical prescription data. The processor instructions further cause the processor to transmit the order to an online pharmacy store server for procuring and dispatching the plurality of medicines to the patient. An automatically generated unique indicia may be allocated to each of the plurality of medicines and a label of each of the unique generated indicia may be affixed to a packaging of each of a plurality of medicines as provided in the medical prescription data.

In an embodiment, the patient provides input via the GUI to confirm a new indicium assignment associated with one or more medicines or edit the indicia assigned to the new medicine. Further, the patient may manually edit the indicia assignment associated with the plurality of medicines at any time. Furthermore, the patient may manually add medicines and supplements that have not been added by a pharmacy that has a connection to the system. The patient may mark one or more medicines from the plurality of medicines as discontinued via the GUI at any time instant. In an embodiment, the medical prescription is received by the electronic device from a doctor.

The processor instructions further cause the processor to provide a reminder notification to the patient to consume the plurality of medicines based on the medical prescription data. In an embodiment, providing the reminder notification at a time instant includes displaying the unique indicia associated with the medicine to be consumed at time instant as prescribed in the medical prescription data. The providing of the notification further includes displaying at least one of the name of the medicine, a visual representation of the medicine, a medical composition and concentration of the medicine, dosage of the medicine, and a time at which the prescribed medication should be consumed, precautionary measures before taking the medicine, or nature of disease for which the medicine is prescribed.

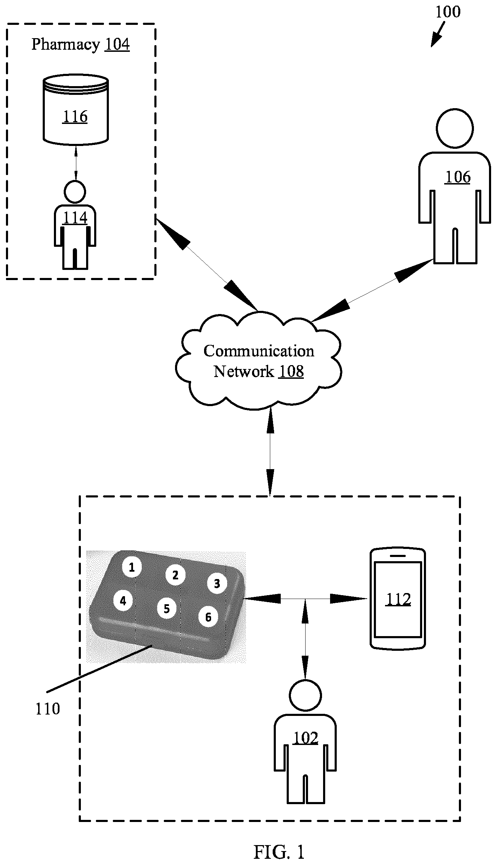

FIG. 1 is a block diagram of an exemplary network environment for managing medicine consumption using a medicine pillbox and an electronic device, in accordance with some embodiments of the present disclosure. Referring now to FIG. 1, an exemplary network environment 100 for for managing medicine consumption using a medicine pillbox and an electronic device is illustrated in accordance with some embodiments of the present disclosure.

The network environment 100 displays a patient 102, a pharmacy 104, and a doctor 106, communicatively coupled to each other via a communication network 108. In an implementation, the patient 102 may be associated with a dedicated medicine pillbox 110 and an electronic device 112. Further, the pharmacy 104 may include a chemist 144 and a pharmacy server 116. Furthermore, the previously mentioned elements may be communicatively coupled with each other via the communication network 108, by virtue of their association with the patient 102 and the pharmacy 104.

A person of ordinary skill in the art will appreciate that for the purpose of illustration, only one user is depicted. However, the network environment 100 may include a plurality of patients, pharmacies, and doctors, communicatively coupled with each other via the communication network 108, without limiting the scope of this disclosure.

The patient 102 may be associated with the medicine pillbox 110 that may include a micro controller unit (MCU), a transceiver installed within the medicine pillbox and configured to communicate with an electronic device 112, a plurality of compartments configured to store a plurality of medicines, and a memory communicatively coupled to the MCU. The plurality of compartments may include compartments 110₁ to 110ₙ. In an implementation, each of the plurality of compartments of the medicine pillbox 110 may be secured closed using a lid associated with each of the compartments from the plurality of compartments 110₁ to 110ₙ, individually. In such an implementation, the lid may include an electronic display for displaying a unique identification for a compartment. The lid may further include allowance for a sticker or a scribble pad, where the unique identification may be provided by the patient 102 or the chemist 114. In another implementation, each of the plurality of compartments 110₁ to 110ₙ of the medicine pillbox 110 may be secured closed using a single lid associated with the medicine pillbox 110. In such an implementation, the identification for each of the plurality of compartments 110₁ to 110ₙ may be viewed after opening the lid of the medicine pillbox 110.

The patient 102 may be associated with the electronic device 112 that may include suitable logic, circuitry, interfaces, and/or code for displaying one or more graphical user interfaces (GUI) of an application that assists the patient 102 in managing the medicine consumption. The GUI may be displayed on a display screen of the electronic device 112. Examples of implementation of the display screen of the electronic devices may include, but are not limited to, a Liquid Crystal Display (LCD) display, a Light Emitting Diode (LED) display, an Organic LED (OLED) display technology.

The pharmacy 104 may include a chemist or pharmacy technician 114 that may correspond to a licensed pharmacist for facilitating the sales and distribution of the medicines. The pharmacy may include a pharmacy server 116 that may refer to a computing node that may be configured to details of the patients 102 and the associated medicine pillboxes and the electronic devices, registered with the pharmacy 104. In an implementation, the pharmacy server 116 may further store the details of the doctor 106 registered with the pharmacy 104. In an implementation, the chemist 114 may include a special purpose operating system specifically configured to perform one or more database operations on the multimedia content. Examples of database operations may include, but are not limited to, Select, Insert, Update, and Delete. In an implementation, the pharmacy server 116 may include hardware that may be configured to perform one or more predetermined operations. In an implementation, the pharmacy server 116 may be realized through various technologies such as, but not limited to, Microsoft^{®} SQL Server, Oracle^{®}, IBM DB2^{®}, Microsoft Access^{®}, PostgreSQL^{®}, MySQL^{®} and SQLite^{®}, and the like.

The communication network 108 may include a medium through which the patient 102, the medicine pillbox 110, the electronic device 112, the pharmacy 104, the chemist 114, the pharmacy server 116, and the doctor 106 present in the network environment 100, may communicate with each other. Examples of the communication network 108 may include, but are not limited to, the Internet, a cloud network, a Wireless Fidelity (Wi-Fi) network, a Wireless Local Area Network (WLAN), a Local Area Network (LAN), a telephone line (POTS), Long Term Evolution (LTE), and/or a Metropolitan Area Network (MAN). Various devices in the exemplary network environment 100 may be configured to connect to the communication network 108, in accordance with various wired and wireless communication protocols. Examples of such wired and wireless communication protocols may include, but are not limited to, Transmission Control Protocol and Internet Protocol (TCP/IP), User Datagram Protocol (UDP), Hypertext Transfer Protocol (HTTP), File Transfer Protocol (FTP), Zigbee, EDGE, infrared (IR), IEEE^{®} 802.11, 802.16, cellular communication protocols, and/or Bluetooth^{®} (BT) communication protocols.

In operation, the patient 102 may register the medicine pillbox 110 with the pharmacy 104 at the time of purchase. The registration may be facilitated by the electronic device 112 based on the GUI of the medicine consumption management application displayed on the electronic device. In an embodiment, the registration may include providing details of the patient 102. The details may include, but are not limited to, patient identification, physical parameters of the patient, medical history of the patient, any known allergies of the patient, preferred modes of payment, details of preferred payment modes of the patient, address, frequently visited doctors, preferred modes of reminders and notifications, authorized electronic devices for accessing the application, details of the associated electronic devices, and one or more emergency contacts. The registration details may be stored at the pharmacy server 116.

In an embodiment, based on the consultation of the patient 102 with the doctor 106, a medical prescription including medical prescription data may be generated by the doctor 106. The medical prescription data may include information about quantity of each of the medicines, dosage of the medicines, and a time at which the prescribed medication should be consumed.

In an embodiment, the medicine pillbox 110 may generate indicia associated with each of the plurality of compartments. Each of the plurality of compartments may be assigned the unique generated indicia. The generation of the indicia includes generating a sequential number starting from '1' up to 'n' based on a number of the plurality of compartments. In an embodiment, the automatically generated indicium may be a unique colour. For example, 'red' colour for the compartment 110₁, 'blue' colour for 'the compartment 110₂, and 'green' colour for the compartment 110ₙ. A person of ordinary skill in the art will appreciate that the generated indicia may include any other form of identification that includes but is not limited to, alphabets, alphanumeric strings, graphics, and the like.

In an embodiment, the assignment of the indicia to a compartment from the plurality of compartments 110₁ to 110ₙ may be a process that is performed in real-time based on requirements. In another embodiment, the indicia may be assigned to the compartments permanently at the time of manufacture. In yet another embodiment, the generation of the indicia may be in real-time, and the assignment may be performed manually by the patient 102, the doctor 106, or the chemist 114.

In an embodiment, the MCU of the medicine pillbox 110 may transmit the generated indicia to the pharmacy server 116. The transmission may further include transmitting the medical prescription data. Based on the transmitted generated indicia and the medicine prescription data, the chemist 114 may label of each of the plurality of compartments 110₁ to 110ₙ with a corresponding generated indicium. The chemist may further affix the indicia to a packaging of each of a plurality of medicines as provided in the medical prescription. For example, a packaging of 'medicine 1' occurring at first position in the medical prescription may be designated an indication '1', indicative of a requirement to place the medicine in the compartment 1 by the patient 102. In another scenario, the same medicine may be designated a colour 'red', indicative of a requirement to place the medicine in the compartment assigned 'red' colour.

In an embodiment, based on the transmitted medical prescription data, the assigned label may be same for medicines that have same chemical composition, and/or same ingredient concentration, even though the manufacturing entity for such medicines may be different. For example, for medicine having paracetamol in the quantity '500mg' may be assigned same label, such as a label '5', even one of the medicines is manufactured by an entity 'ABC' and the other is manufactured by the entity 'DEF'. Accordingly, the chemist 106 may affix the label '5' on both the medicines so that the patient 102 places both the medicines in the compartment having indicium '5'.

Once the package containing the prescribed medicines is received by the patient 102, the patient 102 may store the labelled medicines in accordance with the label affixed on the medicine and the corresponding compartments of the medicine pillbox 110. It is to be noted here that a particular compartment of the medicine pillbox 110 may be configured to store medicines belonging to same type. Subsequently, upon successful storing of the medicine in the compartments, the medicine pillbox 110, in conjunction with the electronic device 112 may generate one or more reminder alerts for the patient 102 for indicating a suitable time for consuming a medicine. The reminder alerts may further notify the patient 102 about the correct dosage of the medicine to be consumed along with metadata of the medicine that includes, but is not limited to, chemical composition of the medicine, prescribed use of the medicine, prescribed dosage information, physical identification of the medicine, and/or one or more warnings associated with the consumption of the medicine.

In an embodiment, in order to track the consumption of the medicines in the medicine pillbox 110, the MCU of the medicine pillbox 110 may identify if one or more medicines from the plurality of medicines has been discontinued based on an input received from the patient 102, via the electronic device 112. Such a scenario may occur when either a duration for consuming a medicine is over, or the patient 102 has been advised by the doctor 106 to discontinue a medicine. In such a scenario, the MCU, based on such an identification, releases an indicium allocated to the one or more discontinued medicines. Released indicia may be allocated to other medicines that are introduced into the prescribed medical regime of the patient 102. For example, when the medicine stored in the compartment 110₁ is discontinued, the indicia '1' may be allocated to a new medicine that may have been introduced into the prescription of the patient 102, by the doctor 106.

In an embodiment, the reassigned indicia may be displayed on the electronic device 112 of the patient 102 and the patient 102 may be asked to confirm the new assignment. In another embodiment, the patient 102 may be notified, via the electronic device 112, about the indicia, which can be reassigned. Basis one or more inputs provided by the patient 102, the indicia may be assigned to medicines.

In an embodiment, each compartment from the plurality of compartments of the medicine pillbox 110 may be integrated with a weight sensor. The MCU may be configured to track the quantity of a medicine in each of the compartments based on the associated sensors. Basis the tracking, and the medical prescription data, the MCU may automatically place an order at a pharmacy 104 for one or more medicines from the plurality of medicines. In another embodiment, the MCU may be configured to automatically place an order at the pharmacy 104 for one or more medicines from the plurality of medicines, based on the medical prescription data and after a pre-defined time interval.

It should be noted that the various components of the system disclosed above may be implemented in programmable hardware devices such as programmable gate arrays, programmable array logic, programmable logic devices, and so forth. Alternatively, the various modules described above may be implemented in software for execution by various types of processors. An identified module of executable code may, for instance, include one or more physical or logical blocks of computer instructions which may, for instance, be organized as an object, procedure, function, engine, or other construct. Nevertheless, the executables of an identified module need not be physically located together, but may include disparate instructions stored in different locations which, when joined logically together, include the module and achieve the stated purpose of the module. Indeed, a module of executable code could be a single instruction, or many instructions, and may even be distributed over several different code segments, among different applications, and across several memory devices.

As will be appreciated by one skilled in the art, a variety of processes may be employed for transmitting data over a communication network 108. For example, the exemplary network environment 100 may transmit data over a communication network 108 by the processes discussed herein. In particular, as will be appreciated by those of ordinary skill in the art, control logic and/or automated routines for performing the techniques and steps described herein may be implemented by the network environment 100, either by hardware, software, or combinations of hardware and software. For example, suitable code may be accessed and executed by the one or more processors on the network environment 100 to perform some or all of the techniques described herein. Similarly, application specific integrated circuits (ASICs) configured to perform some or all of the processes described herein may be included in the one or more processors on the network environment 100.

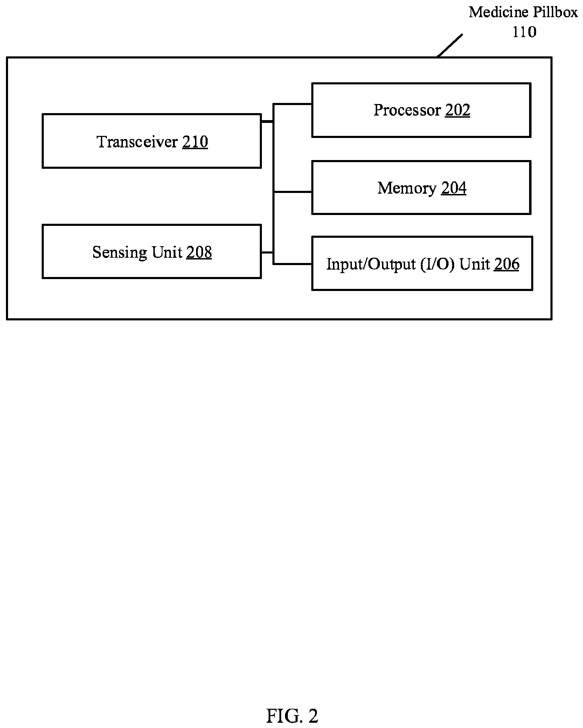

FIG. 2 is a block diagram of exemplary medicine pillbox for managing medicine consumption, in accordance with some embodiments of the present disclosure. FIG. 2 is explained in conjunction with elements from FIG. 1. With reference to FIG. 2, there is shown a medicine pillbox 110 that may include one or more processors, such as a processor 202, a memory 204, an input/output (I/O) unit 206, a sensing unit 208, and/or a transceiver 210. The processor 202 may be communicatively coupled to the memory 204, the I/O unit 206, the sensing unit 208, and/or the transceiver 210. A person of ordinary skill in the art will appreciate that, in an implementation, the aforementioned components may be integrated in a single entity that may constitute a micro controller unit (MCU) of the medicine pillbox 110.

The processor 202 may include suitable logic, circuitry, interfaces, and/or code that may be configured to execute a set of instructions stored in the memory 204. The processor 202 may be configured to assign indicia, track consumption of medicine, and reassign indicia to a new medicine when appropriate, and place order for a medicine at the pharmacy 116. Examples of the processor 202 include, but not limited to, an X86-based processor, a Reduced Instruction Set Computing (RISC) processor, an Application-Specific Integrated Circuit (ASIC) processor, a Complex Instruction Set Computing (CISC) processor, and/or other processor.

The memory 204 may include suitable logic, circuitry, and/or interfaces that may be configured to store a machine code and/or a computer program with at least one code section executable by the processor 202. The memory 204 may be configured to store the indicia generated by the processor 202 for each of the plurality of compartments 110₁ to 110ₙ of the medicine pillbox 110. The memory 204 may be further configured to store the details of the patient 102, such as details of the doctor 106 visited by the patient 102, details of the one or more electronic devices associated with the patient 102, details of the pharmacy from where to order the prescribed medication, and the like. Examples of implementation of the memory 204 may include, but are not limited to, Random Access Memory (RAM), Read Only Memory (ROM), Hard Disk Drive (HDD), and/or a Secure Digital (SD) card.

The I/O unit 206 may include suitable logic, circuitry, interfaces, and/or code that may be configured to render, to the UI objects generated by the processor 202 on the display screen associated with the medicine pillbox 110. The I/O unit 206 may be further configured to render one or more reminder alerts generated by the medicine pillbox 110 to alert the patient 102 about the schedule for consuming the prescribed medicines, and/or about the available quantity of medicines in the medicine pillbox 110. Examples of the display screen may include, but are not limited to, Liquid Crystal Display (LCD) display, Light Emitting Diode (LED) display, Organic LED (OLED) display technology, and/or the like.

In an implementation, for additional security for authenticating an identity of a patient 102, the I/O unit 206 may be further equipped with a photographic optical system, such as a photographic lens and/or a zoom lens, as well as one or more image sensors, such as a charge-coupled device (CCD) or a complementary metal-oxide-semiconductor (CMOS), a digital camera, a camera embedded in a personal digital assistant (PDA), a video camera, and/or a motion camera. A person of ordinary skill in the art will appreciate that the I/O module 206 may further include one or more audio based output devices for enabling communication with a user and an executive.

The sensing unit 208 may include suitable logic, circuitry, interfaces, and/or code that may be configured to keep a track of a quantity of the one or more prescribed medicines available in designated compartments of the medicine pillbox 110. The sensing unit 208 may include one or more weight sensors or load cells for performing the tracking of the quantities. The weight sensors may be configured to detect a change in the quantities of medicines in the compartments of the medicine pillbox and any change beyond a pre-defined threshold may be reported to the processor 202.

The transceiver 210 may include suitable logic, circuitry, interfaces, and/or code that may be configured to facilitate communication among the medicine pillbox 110 and the doctor 106 and the pharmacy 104 along with its associated entities. The transceiver 210 may be implemented based on known technologies to support wired or wireless communication. The transceiver 210 may include, but is not limited to, an antenna, a radio frequency (RF) transceiver, one or more amplifiers, a tuner, one or more oscillators, a digital signal processor, a coder-decoder (CODEC) chipset, a subscriber identity module (SIM) card, and/or a local buffer. The transceiver 210 may communicate via wireless communication with networks, such as the Internet, an Intranet and/or a wireless network, such as a cellular telephone network, a wireless local area network (LAN) and/or a metropolitan area network (MAN). The wireless communication may use any of a plurality of communication standards, protocols and technologies, such as Global System for Mobile Communications (GSM^{®}), Enhanced Data GSM Environment (EDGE^{®}), wideband code division multiple access (W-CDMA^{®}), code division multiple access (CDMA^{®}), Long Term Evolution (LTE^{®}), time division multiple access (TDMA), Bluetooth^{®}, Wireless Fidelity (Wi-Fi^{®}) (such as IEEE^{®} 802.11a, IEEE^{®} 802.11b, IEEE 802.11g and/or IEEE^{®} 802.11n), voice over Internet Protocol (VoIP^{®}), Wi-MAX^{®}, a protocol for email, instant messaging, and/or Short Message Service (SMS).

In operation, the registration of the medicine pillbox 110 with the pharmacy 104 may be performed by the patient 102, at the time of purchase. The registration may be facilitated by the processor 202, in conjunction with the transceiver 210, based on the GUI of the medicine consumption management application displayed on the electronic device. The details of the registration have been explained in FIG. 1.

In an embodiment, based on the consultation of the patient 102 with the doctor 106, a medical prescription including medical prescription data may be generated by the doctor 106. The medical prescription data may include information about quantity of each of the medicines, dosage of the medicines, and a time at which the prescribed medication should be consumed.

Upon generation of the medical prescription, the processor 202 may be configured to synchronize the medical prescription data with the memory 204. Once the medical prescription data is received, the processor 202, in conjunction with I/O unit 206, may be configured to render information of the medical prescription data on an electronic device 112 using the GUI of the medical consumption management application. Once displayed, the patient 102 may provide one or more inputs for placement of an order including one or more medicines prescribed in the medical prescription. The confirmation may be received by the transceiver 210. In another embodiment, the medicine pillbox 110 may be configured to place an order for delivery of the prescribed medicines automatically without any input from the patient 102.

To this end, the processor 202 may be configured to generate an indicium associated with each of the plurality of compartments. Each of the plurality of compartments may be assigned the unique generated indicia. The generation of the indicia includes generating a sequential number starting from '1' up to 'n' based on a number of the plurality of compartments. In an embodiment, the automatically generated indicia may have an associated unique colour. For example, 'red' colour for the compartment 110₁, 'blue' colour for 'the compartment 110₂, and 'green' colour for the compartment 110ₙ. A person of ordinary skill in the art will appreciate that the generated indicia may include any other form of identification that includes but is not limited to, alphabets, alphanumeric strings, graphics, and the like.

The processor 202 may be further configured to assign the generated indicia to a compartment from the plurality of compartments 110₁ to 110ₙ in real-time. In another embodiment, the processor 202 may be further configured to assign the generated indicia to a compartment from the plurality of compartments 110₁ to 110ₙ at the time of registration of the medicine pillbox at the pharmacy server 116.

In an embodiment, the processor 202, in conjunction with the transceiver 210, may be configured to transmit the generated indicia to the pharmacy server 116. The transmission may further include transmitting the medical prescription data. Based on the transmitted generated indicia and the medicine prescription data, the chemist 114 may label of each of the plurality of compartments 110₁ to 110ₙ with a corresponding generated indicia. The chemist may further affix the indicia to a packaging of each of a plurality of medicines as provided in the medical prescription. For example, a packaging of 'medicine 1' occurring at first position in the medical prescription may be designated an indication '1', indicative of a requirement to place the medicine in the compartment 1 by the patient 102. In another scenario, the processor 202 may be configured to designated a colour 'red', indicative of a requirement to place the medicine in the compartment assigned 'red' colour.

Upon receipt of the package containing the prescribed medicines by the patient 102, the processor 202, in conjunction with the electronic device 112, may be configured to instruct the patient 102 for storing the labelled medicines in accordance with the label affixed on the medicine and the corresponding compartments of the medicine pillbox 110. To this end, the processor 202, in conjunction with the electronic device 112, may be configured to render one or more graphical instructions, animations, and/or audio/visual prompts that guide the patient 102 in storing the medicine in designated compartments of the medicine pillbox 110., It is to be noted here that a particular compartment of the medicine pillbox 110 may be configured to store medicines belonging to same type.

Subsequently, upon successful storing of the medicine in the compartments, the processor 202, in conjunction with the I/O unit 206 and/or the electronic device 112, may be configured to generate one or more reminder alerts for the patient 102 for indicating a suitable time for consuming a medicine. The reminder alerts may further notify the patient 102 about the correct dosage of the medicine to be consumed along with metadata of the medicine that includes, but is not limited to, chemical composition of the medicine, prescribed use of the medicine, prescribed dosage information, physical identification of the medicine, and/or one or more warnings associated with the consumption of the medicine.

In an embodiment, in order to track the consumption of the medicines in the medicine pillbox 110, the processor 202, in conjunction with the transceiver 210, based on information communicated by the doctor 106 or the pharmacy server 104, may be configured to identify if one or more medicines from the plurality of medicines has been discontinued based on an input received from the patient 102, via the electronic device 112. Such a scenario may occur when either a duration for consuming a medicine is over, or the patient 102 has been advised by the doctor 106 to discontinue a medicine. In such a scenario, the processor 202 may be configured to release an indicium allocated to the one or more discontinued medicines. The processor 202 may be further configured to allocate the released indicia to other medicines that are introduced into the prescribed medical regime of the patient 102. For example, when the medicine stored in the compartment 110₁ is discontinued, the indicia '1' may be allocated to a new medicine that may have been introduced into the prescription of the patient 102, by the doctor 106.

In an embodiment, the processor 202, in conjunction with the I/O unit 206 and/or the electronic device 112, may be configured to display the reassigned indicia on the electronic device 112 of the patient 102. Based on one or more inputs provided by the patient 102, the processor 202 may be configured to confirm the new assignment and render the reassignment on the display associated with a compartment by use of the I/O unit 206. In another embodiment, the processor 202, in conjunction with the transceiver 210 may be further configured to notify the reassignment to the patient 102, via the electronic device 112. The notification may include information about the indicia which can be reassigned. Basis one or more inputs provided by the patient 102, the indicia may be assigned to medicines.

In an embodiment, the processor 202 in conjunction with the sensing unit 208 may be configured to track the quantity of a medicine in each of the compartment 110₁ to 110ₙ. Basis the tracking, and the medical prescription data, the processor 202 may be configured to automatically place an order at a pharmacy 104 for one or more medicines from the plurality of medicines. In another embodiment, the processor 202 may be configured to automatically place an order at the pharmacy 104 for one or more medicines from the plurality of medicines, based on the medical prescription data and after a pre-defined time interval.

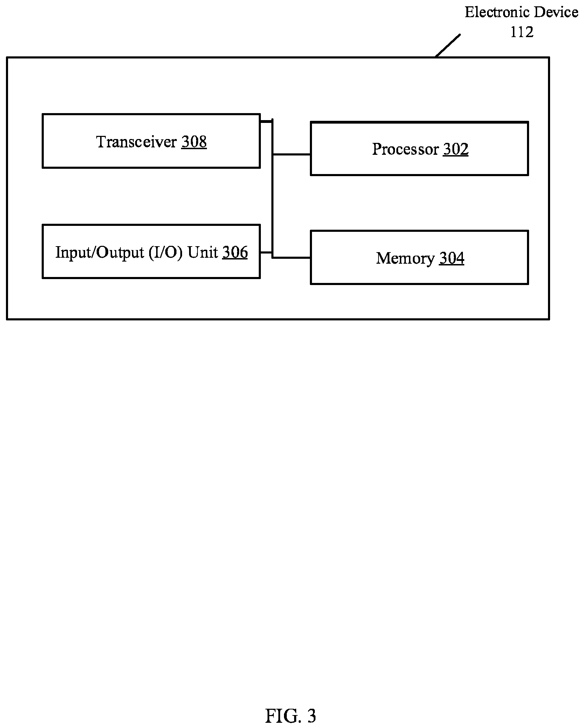

FIG. 3 is a block diagram of exemplary electronic device for managing medicine consumption, in accordance with some embodiments of the present disclosure. FIG. 3 is explained in conjunction with elements from FIGs. 1 and 2. With reference to FIG. 3, there is shown the electronic device 112 that may include one or more processors, such as a processor 302, a memory 304, an input/output (I/O) unit 306, and/or a transceiver 308. The processor 302 may be communicatively coupled to the memory 304, the I/O unit 306, and/or the transceiver 308.

The processor 302 may include suitable logic, circuitry, interfaces, and/or code that may be configured to execute a set of instructions stored in the memory 204. The processor 302 may be configured to receive inputs for booking medicines, receive generated indicia for one or more compartments of a medicine pillbox, place an order for the medicine at a pharmacy, and provide adequate reminder alerts to the patients for consumption of medicines. Examples of the processor 202 include, but not limited to, an X86-based processor, a Reduced Instruction Set Computing (RISC) processor, an Application-Specific Integrated Circuit (ASIC) processor, a Complex Instruction Set Computing (CISC) processor, and/or other processor.

The memory 304 may include suitable logic, circuitry, and/or interfaces that may be configured to store a machine code and/or a computer program with at least one code section executable by the processor 302. The memory 304 may be configured to store the details of the medicine pillbox 110 associated with the patient 102, the indicia generated by the medicine pillbox 110 for each of the plurality of compartments 110₁ to 110ₙ of the medicine pillbox 110, details of the preferred pharmacy of the patient 102, details of preferred mode of reminder alerts, and the like. Examples of implementation of the memory 304 may include, but are not limited to, Random Access Memory (RAM), Read Only Memory (ROM), Hard Disk Drive (HDD), and/or a Secure Digital (SD) card.

The I/O unit 306 may include suitable logic, circuitry, interfaces, and/or code that may be configured to render, to the UI objects generated by the processor 302 on the display screen associated with the electronic device 112. The I/O unit 306 may be further configured to render one or more reminder alerts generated by the electronic device 112 to alert the patient 102 about the schedule for consuming the prescribed medicines, and/or about the available quantity of medicines in the medicine pillbox 110. Examples of the display screen may include, but are not limited to, Liquid Crystal Display (LCD) display, Light Emitting Diode (LED) display, Organic LED (OLED) display technology, and/or the like.

The transceiver 308 may include suitable logic, circuitry, interfaces, and/or code that may be configured to facilitate communication among the electronic device 112, the medicine pillbox 110, and the doctor 106 and the pharmacy 104 along with its associated entities. The transceiver 308 may be implemented based on known technologies to support wired or wireless communication. The transceiver 308 may include, but is not limited to, an antenna, a radio frequency (RF) transceiver, one or more amplifiers, a tuner, one or more oscillators, a digital signal processor, a coder-decoder (CODEC) chipset, a subscriber identity module (SIM) card, and/or a local buffer. The transceiver 308 may communicate via wireless communication with networks, such as the Internet, an Intranet and/or a wireless network, such as a cellular telephone network, a wireless local area network (LAN) and/or a metropolitan area network (MAN). The wireless communication may use any of a plurality of communication standards, protocols and technologies, such as Global System for Mobile Communications (GSM^{®}), Enhanced Data GSM Environment (EDGE^{®}), wideband code division multiple access (W-CDMA^{®}), code division multiple access (CDMA^{®}), Long Term Evolution (LTE^{®}), time division multiple access (TDMA), Bluetooth^{®}, Wireless Fidelity (Wi-Fi^{®}) (such as IEEE^{®} 802.11a, IEEE^{®} 802.11b, IEEE 802.11g and/or IEEE^{®} 802.11n), voice over Internet Protocol (VoIP^{®}), Wi-MAX^{®}, a protocol for email, instant messaging, and/or Short Message Service (SMS).

In operation, the processor 302 in conjunction with the I/O unit 306, may be configured to receive an input via Graphical User Interface (GUI) from the patient 102 for booking an order of a plurality of medicines based on medical prescription data. The processor 302 may be configured to transmit the order to an online pharmacy server 116 for procuring and dispatching the plurality of medicines to the patient 102.

In an embodiment, the processor 302 in conjunction with the transceiver 308, may be configured to receive one or more an automatically generated unique indicia for each of the compartment of the medicine pillbox 110. The processor 302 may be configured to generate a list of medicines that correspond to the medication prescribed to the patient 102 by the doctor 106. In the list, the processor 302 may be configured to assign indicia to each medicine and the assignment is based on the received indicia assignment from the medicine pillbox 110. The processor 302 in conjunction with the transceiver 308 may be further configured to communicate the list of the medicine and the allocated indicia of each of the plurality of medicines, and of the compartments, to the pharmacy server 116. Upon receipt of the aforesaid information at the pharmacy server 116, the chemist may affix each packaging of the medicine in the list with the received unique generated indicia.

In an embodiment, the processor 302 may be configured to provides one or more UI objects on the display screen of the electronic device 112. The UI objects may be provided to the patient 102 in order to receive one or more confirmation inputs that indicate a new indicia assignment associated with one or more medicines or a new medicine. The confirmation inputs may further correspond to an input for editing the indicia assigned to one or more medicines or a new medicine.

Furthermore, the processor 302 in conjunction with the I/O unit may be configured to provide one or more UI objects to the patient 102 so that the patient 102 may manually add medicines and supplements that the patient has not brought from an online pharmacy 104. The processor 302 in conjunction with the I/O unit may be configured to facilitate the patient 102 in marking one or more medicines from the plurality of medicines as discontinued via the one or more UI objects at any time instant.

The processor 302 may be further configured to provide a reminder notification to the patient 102 to consume the plurality of medicines based on the medical prescription data. In an embodiment, providing the reminder notification includes displaying the unique indicia associated with the medicine to be consumed at time instant as prescribed in the medical prescription data. The providing of the notification further includes displaying at least one of the names of the medicine, a visual representation of the medicine, a medical composition and concentration of the medicine, dosage of the medicine, and a time at which the prescribed medication should be consumed, precautionary measures before taking the medicine, or nature of disease for which the medicine is prescribed.

In an embodiment, the processor 302 in conjunction with the I/O unit 306 may be configured to trigger a notification when the patient is not detected within a pre-defined vicinity of the medicine pillbox 110. In an exemplary scenario, the pre-defined vicinity (such as 150-metres) may be user configurable and may be stored in the memory 304. The notification may be rendered on the GUI of the application rendered on the electronic device 112.

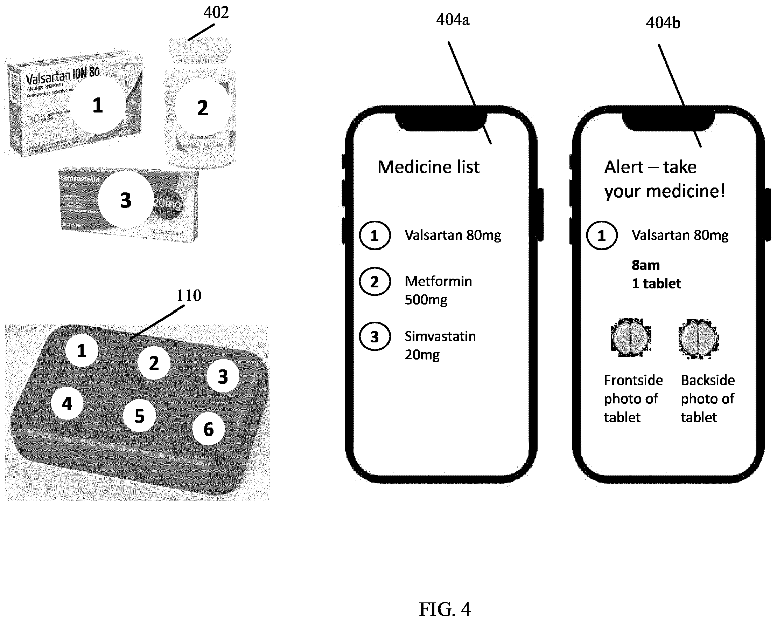

FIG. 4 illustrates an exemplary scenario for managing medicine consumption using a medical pillbox and an electronic device, in accordance with some embodiments of the present disclosure. The elements of FIG. 4 have been described in conjunction with elements of FIG's. 1 to 3.

With reference to FIG. 4, there is shown an exemplary medicine pillbox 110 having a plurality of compartments having indicia '1', '2', and '3'. As explained in FIGs. 1 to 3, the medicine pillbox 110 may automatically generate an indicia associated with each of the plurality of compartments, wherein each of the plurality of compartments is assigned the unique generated indicia. The generated indicia may be displayed, via a display associated with each of the compartment.

The medicine pillbox 110 may transmit the generated indicia to a pharmacy server 116 along with medical prescription data of the medical prescription generated by the doctor 106, for the patient 102. The generated indicia may be translated into a plurality of labels '1', '2', and '3'. Each of the plurality of label of each of the unique generated indicia may be affixed to a packaging of each of a plurality of medicines 302 as provided in the medical prescription. Based on the labelling, it can be ensured by the patient while sorting that each of the plurality of medicines 302 is stored in each of the corresponding plurality of compartments having the same unique generated indicia. For example, the medicine labelled '1' will be stored in the compartment having indicia '1', the medicine labelled '2' will be stored in the compartment having indicia '2', and so on.

Additionally, FIG. 4 further depicts an exemplary interfaces 404a and 404b of a medicine consumption management application. Specifically, the interface 404a depicts the medical prescription data that includes, but is not limited to, names of the prescribed medicines 402, use of the medicines 402, prescribed dosage of the medicines 402, prescribed quantity of consuming each of the medicines 402, and/or a time at which the prescribed medications 402 should be consumed. The interface 404b may depict an interface using which the generated reminder alerts indicating a time when a patient is to consume the plurality of medicines. The interface 404b may further depict the prescribed use of the medicine, prescribed dosage information, physical identification of the medicine, and/or one or more warnings associated with the consumption of the medicine.

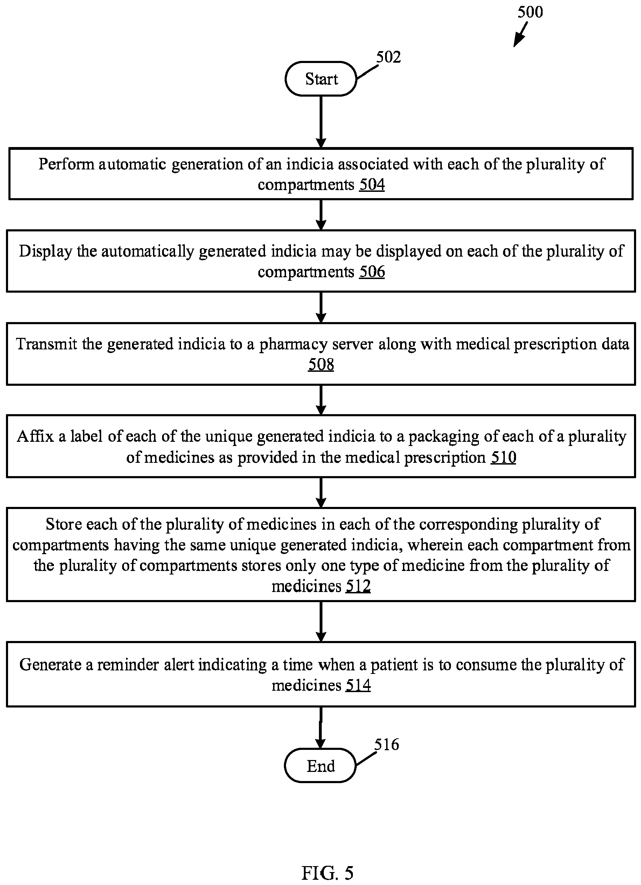

FIG. 5 is a flow diagram of a detailed exemplary process for managing medicine consumption using a medical pillbox and an electronic device, in accordance with some embodiments of the present disclosure. With reference to FIG. 5, there is shown a flow chart 500. The flow chart 500 is described in conjunction with FIG's. 1 to 4. The process starts at step 502 and proceeds to step 504.

At step 504, automatic generation of an indicia associated with each of the plurality of compartments may be performed by the medicine pillbox 110. Each of the plurality of compartments may be assigned the unique generated indicia.

At step 506, the automatically generated indicia may be displayed on each of the plurality of compartments. At step 508, the generated indicia may be transmitted to a pharmacy server 116 along with medical prescription data. At step 510, a label of each of the unique generated indicia may be affixed to a packaging of each of a plurality of medicines as provided in the medical prescription. In an embodiment, the medical prescription data includes information about quantity of each of the medicines, dosage of the medicines, and a time at which the prescribed medication should be consumed. Further, the medical prescription may be received by the medicine pillbox from a doctor.

At step 512, each of the plurality of medicines may be stored in each of the corresponding plurality of compartments having the same unique generated indicia. Further, each compartment from the plurality of compartments stores only one type of medicine from the plurality of medicines. In an embodiment, the patient sorts each of the plurality of medicines in the corresponding compartment by matching the label affixed on the packaging of each of a plurality of medicines with the indicia displayed on each of the plurality of compartments. At step 514, a reminder alert indicating a time when a patient is to consume the plurality of medicines may be generated. The control passes to end step 516.

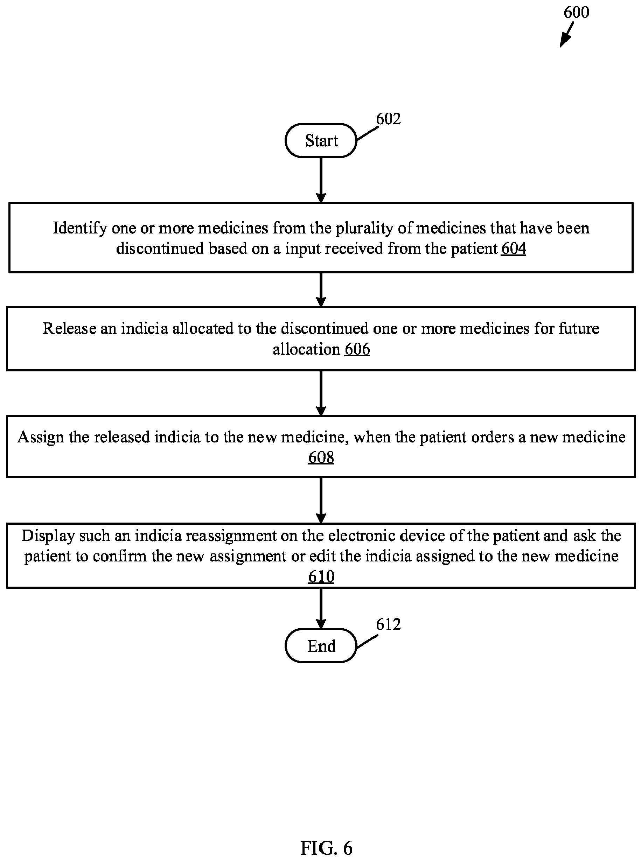

FIG. 6 is a flow diagram of a detailed exemplary process for generating indicia associated with plurality of compartments of a medicine pillbox for managing medicine consumption, in accordance with some embodiments of the present disclosure. With reference to FIG. 6, there is shown a flow chart 600. The flow chart 600 is described in conjunction with FIG's. 1 to 4. The process starts at step 602 and proceeds to step 604.

At step 604, one or more medicines from the plurality of medicines that have been discontinued may be identified based on a input received from the patient 102, via the electronic device 112. At step 606, an indicium allocated to the discontinued one or more medicines may be released for future allocation.

At step 608, when the patient 102 orders a new medicine, then the released indicia may be assigned to the new medicine. At step 610, such an indicia reassignment may be displayed on the electronic device 112 of the patient 102 and the patient may be asked to confirm the new assignment or edit the indicia assigned to the new medicine. The control passes to end step 612.

As will be also appreciated, the above described techniques may take the form of computer or controller implemented processes and apparatuses for practicing those processes. The disclosure can also be embodied in the form of computer program code containing instructions embodied in tangible media, such as floppy diskettes, CD-ROMs, hard drives, or any other computer-readable storage medium, wherein, when the computer program code is loaded into and executed by a computer or controller, the computer becomes an apparatus for practicing the invention. The disclosure may also be embodied in the form of computer program code or signal, for example, whether stored in a storage medium, loaded into and/or executed by a computer or controller, or transmitted over some transmission medium, such as over electrical wiring or cabling, through fiber optics, or via electromagnetic radiation, wherein, when the computer program code is loaded into and executed by a computer, the computer becomes an apparatus for practicing the invention. When implemented on a general-purpose microprocessor, the computer program code segments configure the microprocessor to create specific logic circuits.

As will be appreciated by those skilled in the art, the techniques described in the various embodiments discussed above provides method, device, and medicine pillbox that facilitates seamless integration of a pillbox and peripheral devices that synergistically operate to assist a patient in efficiently managing medicine consumption. The proposed method, device, and medicine obviated the need for arranging the medicines in correct order in various compartments. In addition, the proposed method, device, and medicine pillbox obviates the need for a patient or caretaker to track the quantities of medicines available. The aforesaid features lead to offloading the burden of managing the medicine consumption from the patient or caretaker significantly thereby leading to an efficient and a stress-free method for managing medicine consumption.

The specification has described system and method for managing medicine consumption using a medical pillbox and an electronic device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope and spirit of the disclosed embodiments.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope and spirit of disclosed embodiments being indicated by the following claims.

## Claims

1. A medicine pillbox for managing medicine consumption, the medicine pillbox comprising:
a micro controller unit (MCU);
a transceiver installed within the medicine pillbox and configured to communicate with an electronic device;
a plurality of compartments configured to store a plurality of medicines;
a memory communicatively coupled to the MCU, wherein the memory stores MCU instructions, which, on execution, cause the MCU to:
automatically generate an indicium associated with each of the plurality of compartments, wherein each of the plurality of compartments is assigned the unique generated indicia;
transmit the generated indicia to a pharmacy server along with medical prescription data, wherein a label of each of the unique generated indicia is affixed to a packaging of each of a plurality of medicines as provided in the medical prescription, wherein each of the plurality of medicines is stored in each of the corresponding plurality of compartments having the same unique generated indicia, wherein each compartment from the plurality of compartments stores only one type of medicine from the plurality of medicines; and
generate a reminder alert indicating a time when a patient is to consume the plurality of medicines.

2. The medicine pillbox of claim 1, wherein the medical prescription data comprises information about quantity of each of the medicines, dosage of the medicines, and a time at which the prescribed medication should be consumed.

3. The medicine pillbox of claim 1, wherein automatically generating the indicia comprises generating a sequential number starting from '1' up to 'N' based on a number of the plurality of compartments, wherein the automatically generated indicia is displayed on each of the plurality of compartments.

4. The medicine pillbox of claim 1, wherein the automatically generated indicia has an associated unique colour, wherein pharmacy personnel affixes a label of each of the unique generated indicia with an associated unique colour to a packaging of each of a plurality of medicines as provided in the medical prescription.

5. The medicine pillbox of claim 1, wherein each of the plurality of compartments has a plurality of openable lids.

6. The medicine pillbox of claim 1, wherein the medicine pillbox has an openable lid that covers each of the plurality of compartments.

7. The medicine pillbox of claim 1, further comprising a weight sensor associated with each of the plurality of compartments, wherein the MCU is configured to automatically place an order at a pharmacy store server for one or more medicines from the plurality of medicines based on the medical prescription data and the information received from the weight sensor.

8. The medicine pillbox of claim 1, wherein the MCU is configured to automatically place an order at the pharmacy store server for one or more medicines from the plurality of medicines based on the medical prescription data and after a pre-defined time interval.

9. The medicine pillbox of claim 1, wherein the patient sorts each of the plurality of medicines in the corresponding compartment by matching the label affixed on the packaging of each of a plurality of medicines with the indicia displayed on each of the plurality of compartments.

10. The medicine pillbox of claim 1, wherein automatically generating the indicia associated with each of the plurality of compartments further comprises:
identifying if one or more medicines from the plurality of medicines has been discontinued based on a user input received via the electronic device, wherein based on such identification the indicia allocated to the one or more medicines is released for future allocation, wherein if a user orders a new medicine then the released indicia is assigned to the new medicine, wherein such an indicia reassignment is displayed on the electronic device of the patient and the patient needs to confirm the new assignment or edit the indicia assigned to the new medicine.

11. The medicine pillbox of claim 1, wherein the medical prescription is received by the medicine pillbox from a doctor.

12. An electronic device for managing medicine consumption, the electronic device comprising:
a processor; and
a memory communicatively coupled to the processor, wherein the memory stores processor instructions, which, on execution, causes the processor to:
receive an input via Graphical User Interface (GUI) from a patient to book an order of a plurality of medicines based on medical prescription data;
transmit the order to an online pharmacy store server for procuring and dispatching the plurality of medicines to the patient, wherein an automatically generated unique indicia is allocated to each of the plurality of medicines, wherein a label of each of the unique generated indicia is affixed to a packaging of each of a plurality of medicines as provided in the medical prescription data; and
provide a reminder notification to the patient to consume the plurality of medicines based on the medical prescription data.

13. The electronic device of claim 12, wherein the patient marks one or more medicines from the plurality of medicines as discontinued via the GUI at any time instant.

14. The electronic device of claim 12, wherein the patient provides input via the GUI to confirm a new indicia assignment associated with one or more medicines or edit the indicia assigned to the new medicine.

15. The electronic device of claim 12, wherein the patient can manually edit the indicia assignment associated with the plurality of medicines at any time.

16. The electronic device of claim 12, wherein the patient can manually add medicines and supplements that the patient has not brought from an online pharmacy store.

17. The electronic device of claim 12, wherein providing the reminder notification at a time instant comprises:
displaying the unique indicia associated with the medicine to be consumed at time instant as prescribed in the medical prescription data; and
displaying at least one of: the name of the medicine, a visual representation of the medicine, a medical composition and concentration of the medicine, dosage of the medicine, and a time at which the prescribed medication should be consumed, precautionary measures before taking the medicine, or nature of disease for which the medicine is prescribed.

18. The electronic device of claim 12, wherein the medical prescription is received by the electronic device from a doctor.

19. A method for managing medicine consumption using a medicine pillbox and an electronic device, the method comprising:
automatically generating, by a micro controller unit (MCU), an indicia associated with each of the plurality of compartments of the medicine pillbox, wherein each of the plurality of compartments is assigned the unique generated indicia;
transmitting, by the MCU, the generated indicia to an online pharmacy store server along with medical prescription data, wherein a label of each of the unique generated indicia is affixed to a packaging of each of a plurality of medicines as provided in the medical prescription, wherein each of the plurality of medicines is stored in each of the corresponding plurality of compartments having the same unique generated indicia, wherein each compartment from the plurality of compartments stores only one type of medicine from the plurality of medicines;
generating, by the MCU, a reminder alert indicating a time when a patient is to consume the plurality of medicines; and
displaying, by the MCU, the unique indicia associated with the medicine to be consumed at time instant as prescribed in the medical prescription data.
